# EUROPEAN PATENT SPECIFICATION

(11) **EP 3 506 055 B1**
(45) Date of publication and mention of the grant of the patent: **11.09.2024**
(21) Application number: 17210834.2
(22) Date of filing: 28.12.2017
(51) Int. Cl.: G06F 3/01, G06F 3/038, A61B 3/113

(54) **METHOD FOR EYE-TRACKING CALIBRATION WITH SPLASH SCREEN**
VERFAHREN ZUR AUGENVERFOLGUNGSKALIBRIERUNG MIT STARTBILDSCHIRM
PROCÉDÉ D'ÉTALONNAGE DE SUIVI OCULAIRE COMPORTANT UN ÉCRAN DE DÉMARRAGE

(43) Date of publication of application: 03.07.2019
(73) Proprietor: Vestel Elektronik Sanayi ve Ticaret A.S., 45030 Manisa (TR)
(72) Inventor: KIRISKEN, Barbaros, 45030 Manisa (TR)
(74) Representative: Kehl, Ascherl, Liebhoff & Ettmayr Patentanwälte Partnerschaft mbB

(56) References cited:
- WO-A1-2016/142933
- US-A1- 2014 055 337
- US-A1- 2015 302 585
- CHI JIAN-NAN ET AL: "Key Techniques of Eye Gaze Tracking Based on Pupil Corneal Reflection", INTELLIGENT SYSTEMS, 2009. GCIS '09. WRI GLOBAL CONGRESS ON, IEEE, PISCATAWAY, NJ, USA, 19 May 2009 (2009-05-19), pages 133 - 138, XP031516243, ISBN: 978-0-7695-3571-5

## Description

The present invention refers according to claim 1 to a method for calibrating of an eye-tracking system and according to claim 6 to a method for operating a computer and according to claim 7 to an apparatus for calibration of eye-tracking and according to claim 8 to a computer program product.

### Background of the invention

Document US2014055337 relates to techniques for eye tracking calibration. According to an embodiment, an eye tracking calibration interface may be provided at a computing device. The eye tracking calibration interface may be any configuration of a display screen for performing eye tracking during the startup of an operating system, webpage, application, or other user interface. For instance, an eye tracking calibration interface may designate one or more areas of the display screen as calibration areas. The eye tracking calibration interface may include a message requesting the user to gaze at the calibration areas in order to calibrate eye tracking at the device.

Document WO2014155133 relates to a method for calibrating an eye tracker. According to an embodiment, a user may be asked to look at visual targets, for example, nine dots on a screen arranged in 3 rows and 3 columns. While looking at each visual target, the user is asked to remain still and only move the eyes when looking at each visual target. This procedure is repeated for all visual targets.

Remote eye tracking needs calibration before use. Good calibration can be done by multi point targets shown on the screen. Single point calibration is also possible with recent advancements, with good hardware and large screen and very large UI design such as very large markers. Even single point calibration simplifies calibration process; it is still not intuitive, because someone needs to say to look calibration point and there is a learning curve.

Further documents disclosing tracking techniques are e.g. US2015/302585A1, WO2016/142933A1 or CHI JIAN-NAN ET AL: Key Techniques of Eye Gaze Tracking Based on Pupil Corneal Reflection", INTELLIGENT SYSTEMS, 2009. GCIS '09. WRI GO'LOBAL CONGRESS ON IEEE, PISCATAWAY, NJ, USA 19 MAY 2009, pages 133-138, XP031516243, ISBN: 978-0-7695-3571-5.

### Object of the invention

Thus, it is the object of the present invention to provide an alternative for fast and reliable eye-tracking.

### Summary of the invention

The before mentioned object is solved according to the claims. The present disclosure concerns a method for calibration of eye-tracking. The disclosed method preferably comprises at least the steps: Providing a display of an electronic device and a camera in the field of view of a human being, operating the camera, capturing multiple pictures, in particularly a video sequence, of the human being by means of the camera, displaying a splash screen by means of the display, detecting the eyes of the human being looking at the splash screen by running at least one object detection algorithm, registering the detected eyes as eye reference data and/or calibrating the object detection algorithm by means of the captured pictures and/or by means of the reference data.

Thus, the invention relates to a technique which allows performing calibration for eye tracking of a user based on single point calibration, where a single marker is used as calibration point. As compared to prior art single point calibration techniques, the present technique uses a splash screen of a system or program currently being loaded as the single calibration point and, therefore, calibration is performed in the background while the splash screen is displayed. The technique is based on the assumption that users tend to look at splash screens of loading systems or programs. In this way, eye tracking calibration may be performed without the user noticing it or without explicitly requesting the user to look at the calibration point, respectively. Further preferred embodiments are subject-matter of the following specification parts and/or of the dependent claims.

According to the invention in a predefined time interval the splash screen is rearranged in at least one further position, wherein the eyes of the human being are detected in dependency of the further position of the splash screen. This embodiment is beneficial since multiple different orientations of the eyes can be analyzed, thus the overall calibration is more reliable.

The splash screen comprises according to a further preferred embodiment of the present disclosure a dynamic section, wherein the dynamic section moves within the splash screen and/or wherein the dynamic section comprises an animated element or a movie sequence.

This embodiment is beneficial since due to the dynamic section user interest is increased and thus it is more likely that the user looks onto the screen, in particularly into the splash screen.

The dynamic section is according to a further preferred embodiment of the present disclosure linked to an internet source and displays user related or predefined content, wherein the content is downloaded or streamed. This embodiment is beneficial since the displayed content fits more to the interests of the user thus it is even more likely that the user looks onto the screen, in particularly into the splash screen.

User data identifying interests of the current user is according to a further preferred embodiment of the present disclosure derived from a web profile of said user, of a combination of sex and/or age and/or mood of the user, of browser data and/or of specific user data inputted by the current user. This embodiment is beneficial since the user data can be provided automatically and close to the interests of the user.

According to a further preferred embodiment of the present disclosure the method further comprises the steps of removing the splash screen from the display, determining if the eyes of the human being are detected after the splash screen is removed and performing a multi-point calibration in case the eyes are not detected. It is further possible that the booting respectively loading time of the respective system or program is extended. This embodiment is beneficial since it is assured that the eye-tracking calibration will be done.

The detection algorithm analyzes according to a further preferred embodiment of the present disclosure a pupil center cornea reflection. This embodiment is beneficial since pupil center cornea reflection can be done in a very reliable and fast manner.

The eye reference data comprises according to a further preferred embodiment of the present disclosure data about the orientation of the eyes and/or of the gaze of the human being. This embodiment is beneficial since the respective program or system utilizing a later eye-tracking can be equipped with more complex features.

The splash screen belongs according to a further preferred embodiment of the present disclosure to a program or system started as result of an interaction between the user and the electronic device, wherein the operation of the camera is triggered at the same time when the program or system is selected or started. This embodiment is beneficial since the eye-tracking calibration can be performed before the system or program can be used respectively is ready. Thus, the user comfort is further increased.
- Fig. 1: a shows exemplarily a user looking onto a screen, wherein a splash screen is displayed on that screen;
- Fig. 1b: a further example of a splash screen,
- Fig 2a: an example for a single point calibration,
- Fig. 2b: an example for a multi-point calibration,
- Fig. 2c: a switch from the disclosed splash screen calibration to a single point or multi point calibration.

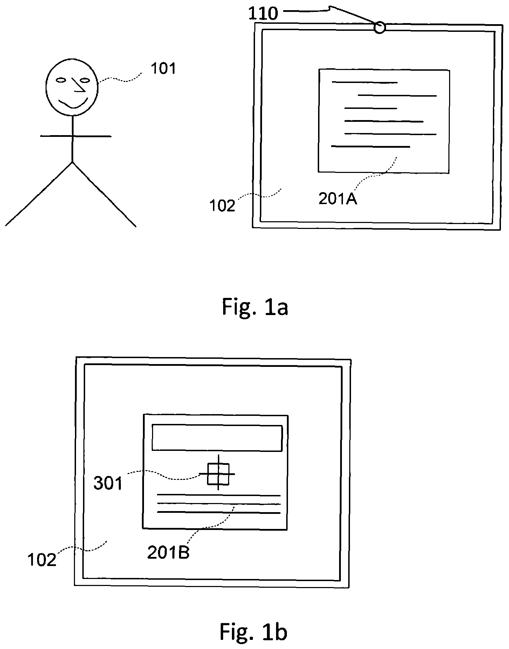

Fig. 1a shows a user 101 looking onto a display 102, wherein a splash screen 201A is displayed onto the display 102. The splash screen 201A belongs to a program or system that starts. With the start or the selection of the program or system an eye-tracking starts, wherein at least one camera 110 tracks the head, in particularly the eyes, of the user 101. The camera 110 can be part of the display 102 respectively of the device having said display or can be an external camera which is connected by means of data transfer with a processing unit that operates the device.

Fig. 1b shows a further example of a splash screen 201B. This splash screen comprises a special marker 301. This special marker can be a predefined image or logo or symbol or a dynamic section within the splash screen 201B.

It is also possible that the user 101 preferably receives an information to look onto the splash screen 201A or 201B. Such an information can be outputted acoustically and/or visually.

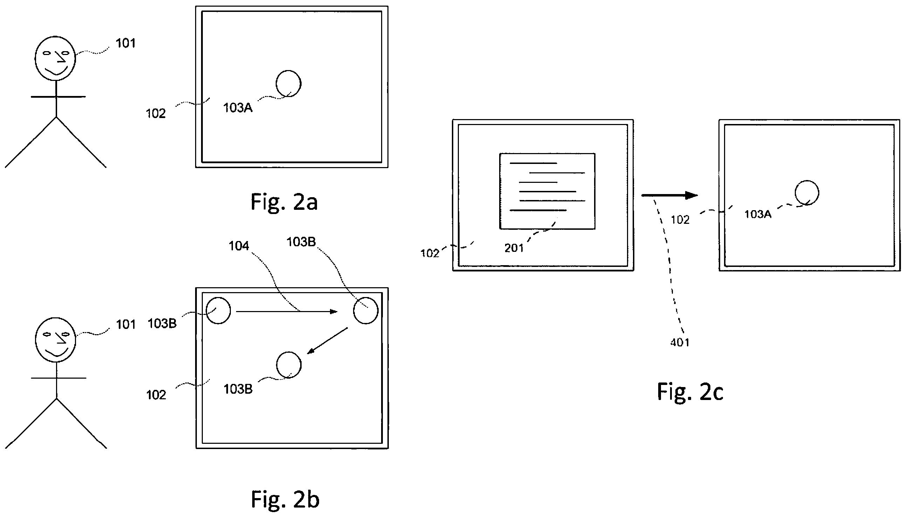

Fig. 2a and fig. 2b show further additional steps, which can be performed after the splash screen eye tracking was done. Fig. 2a shows a single point calibration 103A. The user 101 preferably receives an information to look onto the single-point 103A. Fig. 2b shows a multi-point calibration 103B. The user 101 preferably receives an information to look onto the multi-point 103B. The multi-point system might display multiple points at the same time or one point at different positions (reference number 104) or multiple points at different positions. The camera 110 tracks in each case the movements of the eyes, in particularly the movements of the pupils.

Fig. 2c (reference number 401) indicates that a single-point or multi-point calibration can be performed after a calibration using a splash screen 201 was performed.

Thus, the present disclosure refers to a method for calibration of eye-tracking. The method preferably comprises at least the steps: Providing a display of an electronic device and a camera in the field of view of a human being, operating the camera, capturing multiple pictures, in particularly a video sequence, of the human being by means of the camera, displaying a splash screen by means of the display, detecting the eyes of the human being looking at the splash screen by running at least one object detection algorithm, registering the detected eyes as eye reference data.

Present disclosure has single point calibration for eye tracking, in particularly utilizing algorithm/s similar or identical to the algorithm/s used in prior art. However, it uses splash screen as a calibration point instead of special marker. Splash screen in the disclosure is designed remarkable and also users tend to look on splash screens. When system/program etc. loading splash screen appears at that time user looks onto the splash screen or into the direction of the splash screen calibration is done in the background. With this novel method user feels nearly calibration-less remote eye tracking. With another embodiment program loading time is extended if calibration is not finished.

If the user does not look at the splash screen or calibration cannot be done for any reason then the system shows a normal calibration marker. Splash screen can be a program loading splash screen or bios logo etc.

Therefore, the present disclosure proposes a calibration system for eye tracking devices with at least one screen. The preferred eye tracking system is more likely a PCCR (pupil centre cornea reflection) system.

### List of reference numbers

- 101: User looking to screen
- 102: Screen
- 103: Calibration marker, Calibration point or look marker
- 103A: Single point calibration
- 103B: Multi point calibration
- 104: Marker moves during multi point calibration
- 201A: Splash screen
- 201B: Alternative splash screen with special marker
- 301: Special marker to get user attention
- 401: If system is not able to calibrate over splash screen for any reason, normal calibration starts.

## Claims

1. Method for calibration of eye-tracking,
comprising the steps of:
Providing a display (102) of an electronic device and a camera (110) in the field of view of a human being (101),
Operating the camera (110),
Capturing multiple pictures, in particularly a video sequence, of the human being (101) by means of the camera (110),
wherein the method for calibration of eye-tracking is **characterized in that** it further comprises the steps of:
Displaying a splash screen (201A, 201B) by means of the display (102),
Detecting the eyes of the human being (101) looking at the splash screen (201A, 201B) by running at least one object detection algorithm,
wherein in a predefined time interval the splash screen (201A, 201B) is rearranged in at least one further position, and wherein the eyes of the human being (101) are detected in dependency of the further position of the splash screen (201A, 201B),
Registering the detected eyes as eye reference data, wherein the eye reference data comprises data about the orientation of the eyes of the human being (101).

2. Method according to claim 1,
**characterized in that**
the splash screen (201A, 201B) comprises a dynamic section, wherein the dynamic section moves within the splash screen (201A, 201B), wherein the dynamic section is an animated element or a movie sequence.

3. Method according to claim 2,
**characterized in that**
the dynamic section is linked to an internet source and displays user related or predefined content, wherein the content is downloaded or streamed.

4. Method according to claim 3,
**characterized in that**
the user related or predefined content data identifying interests of the current user and being derived from a web profile of said user, from a combination of sex and/or age of the user, from browser data and/or from specific user data inputted by the current user.

5. Method according to any of the preceding claims
**characterized in that**
the splash screen (201A, 201B) belongs to a program started as result of an interaction between the user and the electronic device,
wherein the operation of the camera (110) is triggered at the same time when the program is selected or started.

6. Method for operating a computer system,
comprising the steps:
Performing predefined operations in dependency of eye positions of the human being (101), wherein the eye positions are validated in dependency of eye reference data captured according to a preceding method claim.

7. An apparatus for calibration of eye-tracking, the apparatus comprising a camera (110) and a display (102), the apparatus further comprising means adapted to execute the steps of method claims 1-6.

8. A Computer program product comprising instructions which, when the program is executed the apparatus of claim 7, cause the apparatus carry out the steps of the method of any of claims 1 to 6.

## Patentansprüche

1. Verfahren zur Kalibrierung von Eye-Tracking,
das die folgenden Schritte aufweist:
Vorsehen eines Displays (102) einer elektronischen Vorrichtung und einer Kamera (110) im Blickfeld eines Menschen (101),
Betreiben der Kamera (110),
Aufnehmen mehrerer Bilder, insbesondere einer Videosequenz, des Menschen (101) mittels der Kamera (110),
wobei das Verfahren zur Kalibrierung des Eye-Trackings **dadurch gekennzeichnet ist, dass** es ferner die folgenden Schritte umfasst:
Anzeigen eines Splash-Screens (201A, 201B) mittels des Displays (102),
Erkennen der Augen des Menschen (101), der auf den Splash-Screen (201A, 201B) schaut, durch Ausführen mindestens eines Objekterkennungsalgorithmus,
wobei in einem vordefinierten Zeitintervall der Splash-Screen (201A, 201B) in mindestens einer weiteren Position neu angeordnet wird, und wobei die Augen des Menschen (101) in Abhängigkeit von der weiteren Position des Splash-Screens (201A, 201B) erfasst werden,
Registrieren der erfassten Augen als Augenreferenzdaten, wobei die Augenreferenzdaten Daten über die Orientierung der Augen des Menschen (101) aufweisen.

2. Verfahren gemäß Anspruch 1,
**dadurch gekennzeichnet, dass**
der Splash-Screen (201A, 201B) einen dynamischen Bereich aufweist, wobei sich der dynamische Bereich innerhalb des Splash-Screens (201A, 201B) bewegt, wobei der dynamische Bereich ein animiertes Element oder eine Filmsequenz ist.

3. Verfahren gemäß Anspruch 2,
**dadurch gekennzeichnet, dass**
der dynamische Bereich mit einer Internetquelle verknüpft ist und benutzerbezogenen oder vordefinierten Inhalt anzeigt, wobei der Inhalt heruntergeladen oder gestreamt wird.

4. Verfahren gemäß Anspruch 3,
**dadurch gekennzeichnet, dass**
die benutzerbezogenen oder vordefinierten Inhaltsdaten Interessen des aktuellen Benutzers identifizieren und aus einem Webprofil des Benutzers, aus einer Kombination von Geschlecht und/oder Alter des Benutzers, aus Browserdaten und/oder aus spezifischen Benutzerdaten, die von dem aktuellen Benutzer eingegeben wurden, abgeleitet werden.

5. Verfahren gemäß einem der vorangehenden Ansprüche
**dadurch gekennzeichnet, dass**
der Splash-Screen (201A, 201B) zu einem Programm gehört, das als Ergebnis einer Interaktion zwischen dem Benutzer und der elektronischen Vorrichtung gestartet wird,
wobei der Betrieb der Kamera (110) zur gleichen Zeit ausgelöst wird, wenn das Programm ausgewählt oder gestartet wird.

6. Verfahren zum Betrieb eines Computersystems,
das die Schritte aufweist:
Durchführen von vordefinierten Operationen in Abhängigkeit von Augenpositionen des Menschen (101), wobei die Augenpositionen in Abhängigkeit von gemäß einem vorangehenden Anspruch erfassten Augenreferenzdaten validiert werden.

7. Vorrichtung zur Kalibrierung von Eye-Tracking, wobei die Vorrichtung eine Kamera (110) und ein Display (102) aufweist, wobei die Vorrichtung ferner Mittel aufweist, die dazu angepasst sind, die Schritte der Verfahrensansprüche 1-6 auszuführen.

8. Computerprogrammprodukt, das Anweisungen aufweist, die, wenn das Programm in der Vorrichtung nach Anspruch 7 ausgeführt wird, die Vorrichtung veranlassen, die Schritte des Verfahrens gemäß einem der Ansprüche 1 bis 6 ausführt.

## Revendications

1. Procédé d'étalonnage de l'eye-tracking,
qui comprend les étapes suivantes :
Fournir un affichage (102) d'un dispositif électronique et une caméra (110) dans le champ de vision d'un être humain (101),
Faire fonctionner la caméra (110),
Enregistrement de plusieurs images, en particulier d'une séquence vidéo, de l'être humain (101) au moyen de la caméra (110),
le procédé d'étalonnage de l'eye-tracking étant **caractérisé en ce qu'**il comprend en outre les étapes suivantes :
Affichage d'un écran d'éclats (201A, 201B) au moyen de l'écran (102),
reconnaître les yeux de la personne (101) qui regarde l'écran d'éclaboussement (201A, 201B) en exécutant au moins un algorithme de reconnaissance d'objet,
dans lequel, à un intervalle de temps prédéfini, le splash-screen (201A, 201B) est réarrangé dans au moins une autre position, et dans lequel les yeux de la personne (101) sont détectés en fonction de l'autre position du splash-screen (201A, 201B),
Enregistrer les yeux détectés en tant que données de référence des yeux, les données de référence des yeux comprenant des données relatives à l'orientation des yeux de l'être humain (101).

2. Procédé selon la revendication 1,
**caractérisé en ce que**
l'écran d'éclaboussement (201A, 201B) comprend une zone dynamique, la zone dynamique se déplaçant à l'intérieur de l'écran d'éclaboussement (201A, 201B), la zone dynamique étant un élément animé ou une séquence de film.

3. Procédé selon la revendication 2,
**caractérisé en ce que**
la zone dynamique est associée à une source Internet et affiche un contenu lié à l'utilisateur ou prédéfini, le contenu étant téléchargé ou diffusé en continu.

4. Procédé selon la revendication 3,
**caractérisé en ce que**
les données de contenu liées à l'utilisateur ou prédéfinies identifient les intérêts de l'utilisateur actuel et sont dérivées d'un profil web de l'utilisateur, d'une combinaison du sexe et/ou de l'âge de l'utilisateur, de données de navigateur et/ou de données d'utilisateur spécifiques entrées par l'utilisateur actuel.

5. Procédé selon l'une des revendications précédentes.
**caractérisé en ce que**
l'écran d'éclaboussement (201A, 201B) fait partie d'un programme qui est lancé à la suite d'une interaction entre l'utilisateur et le dispositif électronique,
dans lequel le fonctionnement de la caméra (110) est déclenché en même temps que le programme est sélectionné ou lancé.

6. Procédé de fonctionnement d'un système informatique,
qui comprend les étapes consistant à :
Exécution d'opérations prédéfinies en fonction de positions oculaires de l'être humain (101), les positions oculaires étant validées en fonction de données de référence oculaires acquises conformément à une revendication précédente.

7. Dispositif d'étalonnage de l'eye-tracking, le dispositif comprenant une
caméra (110) et un écran d'affichage (102), le dispositif comprenant en outre des moyens adaptés pour exécuter les étapes des revendications de procédé 1-6.

8. Produit de programme d'ordinateur comprenant des instructions qui, lorsque le programme est exécuté dans le dispositif selon la revendication 7, font que le dispositif exécute les étapes du procédé selon l'une quelconque des revendications 1 à 6.
